# EUROPÄISCHE PATENTANMELDUNG

(11) **EP 1 421 911 A1**
(43) Veröffentlichungstag der Anmeldung: **26.05.2004**
(21) Anmeldenummer: 03025456.9
(22) Anmeldetag: 05.11.2003
(51) Int. Cl.: A61B 18/14, A61B 17/28

(54) **Chirurgisches Instrument**

(30) Priorität: 19.11.2002 DE 10253760
(71) Anmelder: Aesculap AG & Co. KG, 78532 Tuttlingen (DE)
(72) Erfinder: Rothweiler, Christoph, 78166 Donaueschingen (DE); Weisshaupt, Dieter, 78194 Immendingen (DE)
(74) Vertreter: Hoeger, Stellrecht & Partner

(57) **Zusammenfassung**

Um ein chirurgisches Instrument mit mindestens zwei aneinander oder relativ zueinander bewegbaren Branchen zum Betätigen von Werkzeugen an einem distalen Ende des Instrumentes, wobei die Branchen jeweils einen in einem proximalen Bereich der Branchen abstehenden Haltering tragen, so zu verbessern, daß es jederzeit einfach und sicher von einem Anwender ergriffen werden kann, wird vorgeschlagen, daß mindestens eine der beiden Branchen ein Verbindungselement umfaßt zum Herstellen einer zusätzlichen Verbindung des Halterings mit der Branche und daß das Verbindungselement eine von der Branche weg weisende Haltefläche umfaßt.

## Beschreibung

Die Erfindung betrifft ein chirurgisches Instrument mit mindestens zwei aneinander oder relativ zueinander bewegbaren Branchen zum Betätigen von Werkzeugen an einem distalen Ende des Instrumentes, wobei die Branchen jeweils einen in einem proximalen Bereich der Branchen abstehenden Haltering tragen.

Instrumente der eingangs beschriebenen Art sind in zahlreicher Weise bekannt, beispielsweise als herkömmliche Scheren mit zwei aneinander schwenkbar gelagerten Branchen, welche an ihren distalen Enden Scherenblätter mit eingelagerten Schneiden tragen. Ferner gibt es auch Rohrschaftinstrumente, die quer zur Längsrichtung des Rohrschafts abstehende, relativ zueinander verschwenkbare Branchen aufweisen. Zum Greifen der Branchen des Instruments sind an den Branchen Halteringe angeordnet, durch deren Öffnung Finger eines Anwenders hindurchgestreckt werden können, und zwar, in Abhängigkeit von der Größe des Halterings, ein oder mehrere Finger.

Durch die Anordnung der Halteringe an den Branchen ist ein Griffbereich für einen Anwender genau vorgegeben, was sich häufig als nachteilig erweist.

Daher ist es Aufgabe der vorliegenden Erfindung, ein chirurgisches Instrument der eingangs beschriebenen Art so zu verbessern, daß es jederzeit einfach und sicher von einem Anwender ergriffen werden kann.

Diese Augabe wird bei einen chirurgischen Instrument der eingangs beschriebenen Art erfindungsgemäß dadurch gelöst, daß mindestens eine der beiden Branchen ein Verbindungselement umfaßt zum Herstellen einer zusätzlichen Verbindung des Halterings mit der Branche und daß das Verbindungselement eine von der Branche weg weisende Haltefläche umfaßt.

Der Haltering ist bei bisher bekannten Instrumenten mit der zugehörigen Branche verbunden. Um eine solche Verbindung zu stabilisieren, ist das Verbindungselement nützlich, denn dieses verbindet den Haltering zusätzlich mit der Branche. Ferner wird durch das Verbindungselement eine Haltefläche gebildet, an der sich ein oder mehrere Finger eines Anwenders abstützen oder mit diesen das Instrument halten können. Es ergeben sich somit eine Vielzahl unterschiedlicher Greifmöglichkeiten des Instruments für den Anwender. Beispielsweise lassen sich durch Greifen der Branchen an unterschiedlichen Stellen, das heißt beispielsweise entweder an der Haltefläche oder am Haltering, gezielt Hebelverhältnisse variieren. Dadurch kann das Instrument vom Anwender stets sicher und in gewünschter Weise ergriffen werden.

Um eine besonders flache Bauform des Instruments zu erreichen, ist es günstig, wenn die Halteringe der beiden Branchen seitlich von den Branchen abstehen und eine gemeinsame Ebene definieren.

Damit das Instrument einen Griffbereich ohne Kanten und Ecken aufweist, ist es von Vorteil, wenn das Verbindungselement tangential in den Haltering übergeht.

Um möglichst kantenfreie Außenkonturen zu schaffen, ist bei einer besonders bevorzugten Ausführungsform der Erfindung vorgesehen, daß die Haltefläche tangential in eine von der anderen Branche weg weisende Fläche der Branche übergeht.

Vorzugsweise geht das Verbindungselement tangential in die Branche über. Dies verhindert, daß durch das Verbindungselement beim Verwenden des Instruments Verletzungen weder beim Anwender noch bei einem Patient verursacht werden können.

Vorzugweise geht die Haltefläche tangential in eine radial nach außen weisende Fläche des Halterings über. Dadurch läßt sich der Haltering mit dem daran angeordneten Verbindungselement völlig kantenfrei ausbilden.

Damit ein Abrutschen eines Fingers des Anwenders von der Haltefläche vermieden wird, wird vorgeschlagen, daß der Krümmungsradius der nach außen weisenden Haltefläche mindestens 1 cm, vorzugsweise mindestens 2 cm beträgt.

Auf einfache Weise läßt sich ein durch die Branche definierter Hebelarm verkleinern, wenn sich die Haltefläche vom Haltering in distaler Richtung der Branche erstreckt und wenn der Anwender das Instrument an der Haltefläche ergreift.

Eine besonders vorteilhafte Ergonomie des Instruments ergibt sich, wenn die Haltefläche am Haltering ansetzt in einem Abstand von der Branche, der etwa der halben Quererstreckung des Halterings quer zur Längsrichtung der Branche entspricht.

Eine besonders günstige Form des Griffbereichs des Instruments ergibt sich, wenn die Haltefläche einen von dem Haltering zunehmenden Krümmungsradius aufweist.

Das Gewicht des Instruments läßt sich verringern, wenn das Verbindungselement einen Stützsteg umfaßt. Durch den Stützsteg wird gleichzeitig eine Durchbrechung gebildet, die begrenzt wird durch den Stützsteg, die Branche und den Haltering. Außerdem läßt sich durch Ausbildung eines Stützstegs Material sparen.

Aus Stabilitätsgründen ist es vorteihaft, wenn der Stützsteg eine Querschnittsfläche aufweist, die im wesentlichen der Querschnittsfläche des Halterings entspricht.

Um die Stabilität des Instruments zu erhöhen, kann vorgesehen sein, daß das Verbindungselement einen von dem Haltering, der Branche und der Haltefläche umgebenen Stützbereich ausfüllt. Durch diese massive Ausgestaltung des Griffbereichs wird eine besonders gute Verbindung zwischen dem Haltering und der Branche hergestellt. Das Halten des Instruments wird dadurch erleichtert.

Um Gewicht und Material zu sparen, ist es von Vorteil, wenn das Verbindungselement eine Ausnehmung oder Durchbrechung umfaßt. Außerdem ermöglicht es diese Ausgestaltung, daß auch Finger durch diese Durchbrechung hindurchgestreckt oder in eine Ausnehmung eintauchen können. Ferner lassen sich zusätzliche Bauelemente in vorteilhafter Weise an der Ausnehmung oder in der Durchbrechung anordnen und/oder befestigen.

Ein besonders einfacher Aufbau des Instruments ergibt sich, wenn die Branchen aneinander schwenkbar gelagert sind und wenn die Werkzeuge starr mit den Branchen verbunden sind. Beispielsweise bei herkömmlichen Scheren oder Zangen hat sich eine derartige Ausgestaltung in vorteilhafter Weise bewährt.

Grundsätzlich wäre es denkbar, mit dem Instrument aufgrund einer Relativbewegung der Werkzeuge einen chirurgischen Eingriff durchzuführen. Gemäß einer bevorzugten Ausführungsform der Erfindung kann jedoch vorgesehen sein, daß das Instrument eine elektrische Anschlußvorrichtung umfaßt. Damit läßt es sich mit einer elektrischen Energieversorgung verbinden, beispielsweise einem Hochfrequenzgenerator, so daß mit dem Instrument hochfrequenzchirurgische Eingriffe vorgenommen werden können. Beispielsweise ist es mit einem derartigen Instrument möglich, gleichzeitig zu schneiden und durchtrenntes Gewebe zu koagulieren.

Günstig ist es, wenn die elektrische Anschlußvorrichtung eine Verdrehsicherung umfaßt zum Verhindern eines Verdrehens eines mit der Anschlußvorrichtung verbundenen Anschlußelementes. Beispielsweise kann das elektrisches Anschlußelement in Form eines Steckers mit der Anschlußvorrichtung verbunden werden, ein Verdrehen des Anschlußelements an oder in der Anschlußvorrichtung ist aufgrund der Verdrehsicherung jedoch nicht möglich.

Ein besonders einfacher Aufbau der Verdrehsicherung ergibt sich, wenn die Verdrehsicherung einen mindestens einen Anschlußkontakt umgebende Ausnehmung umfaßt, und wenn die Ausnehmung einen vieleckigen Querschnitt, inbesondere einen 6-eckigen Querschnitt aufweist. Ist das Steckelement korrespondierend zur Ausnehmung ausgebildet, so ergibt sich eine formschlüssige Verbindung zwischen dem Anschlußelement und der Ausnehmung, die ein Verdrehen des Anschlußelements in der Anschlußvorrichtung verhindert.

Denkbar wäre es auch, eine Anschlußvorrichtung als vieleckigen Vorsprung auszugestalten und das Anschlußelement entsprechend als eine im Querschnitt vieleckige Ausnehmung.

Um eine Behinderung des Anwenders durch Anschlußleitungen bei einem operativen Eingriff zu vermeiden, weist der mindestens eine Anschlußkontakt in eine zur Längsrichtung der Branche parallele Richtung. Bevorzugt weist der Anschlußkontakt in proximaler Richtung der Branche, so daß Anschlußleitungen von proximal her kommend an das Instrument herangeführt werden können.

Denkbar wäre es, dem Instrument außer elektrischer Energie auch andere Medien zu- oder diese vom Instrument abzuführen. Zu diesem Zweck ist es vorteilhaft, wenn das Instrument ein Schaltelement umfaßt zum Unterbrechen eines Energieflusses von einer Energiequelle zum Instrument. Beispielsweise ließen sich mit einem derartigen Schaltelement Hochfrequenz-Ströme unterbrechen, aber auch ein Zufluß von Spülflüssigkeit oder eine Absaugvorrichtung abschalten.

Vorteilhaft ist es, wenn das Schaltelement mit dem Instrument lösbar verbindbar ist. Wird für das Instrument keine zusätzliche Energie benötigt, kann das Schaltelement auf einfache Weise vom Instrument entfernt werden. Wird zusätzliche Energie benötigt, läßt es sich wieder mit dem Instrument verbinden.

Eine besonders einfache und sichere Verbindung des Schaltelements mit dem Instrument ergibt sich, wenn das Schaltelement mit dem Instrument verrastbar oder an dieses anclipbar ist.

Um eine besonders gute und definierte Verbindung zwischen dem Schaltelement und dem Instrument zu erreichen, umfaßt das Instrument eine Schaltelementaufnahme. In eine derartige Schaltelementaufnahme kann das Schaltelement eingesetzt werden, beispielsweise form- oder kraftschlüssig. Darüber hinaus kann es zusätzlich am Instrument gesichert werden, beispielsweise durch Verrasten.

Ein besonders einfacher Aufbau des Instruments ergibt sich dann, wenn die Schaltelementaufnahme durch die Ausnehmung oder Durchbrechung des Verbindungselementes gebildet ist. Die Ausnehmung oder Durchbrechung übernimmt damit zwei Funktionen. Einerseits kann sie dazu dienen, daß Finger in sie eingeführt oder durchgeführt werden können, andererseits läßt sich an der Ausnehmung oder Durchbrechung das Schaltelement anbringen.

Günstig ist es, wenn die Werkzeuge Klemmbacken oder Scheren blätter mit Schneiden umfassen. Damit lassen sich auf einfache Weise Zangen oder Scheren bilden.

Besonders ergonomisch läßt sich ein Griffbereich ausbilden, wenn der Haltering rund oder oval ausgeformt ist. Denkbar wäre es auch, den Haltering rechteckig oder in sonstiger Weise vieleckig auszubilden.

Besonders leicht wird das Instrument, wenn es aus Kunststoff hergestellt ist. Außerdem läßt es sich dann besonders einfach und kostengünstig herstellen.

Um eine Stabilität des Instruments zusätzlich zu erhöhen, ist es von Vorteil, wenn die Branche, der Haltering und des Verbindungselement einstückig ausgebildet sind.

Die nachfolgende Beschreibung bevorzugter Ausführungsformen der Erfindung dient im Zusammenhang mit der Zeichnung der näheren Erläuterung. Es zeigen:
- Figur 1:: eine Seitenansicht einer erfindungsgemäßen Schere;
- Figur 2:: ein Griffbereich eines zweiten Ausführungsbeispiels einer Schere;
- Figur 3:: ein Griffbereich eines dritten Ausführungsbeispiels einer Schere;
- Figur 4:: ein Griffbereich eines vierten Ausführungsbeispiels einer Schere;
- Figur 5:: eine Seitenansicht eines fünften Ausführungsbeispiels einer Schere mit Anschlußkontakten;
- Figur 6:: das in Figur 5 dargestellte Instrument mit daran angeordnetem Schaltelement;
- Figur 7:: eine Ansicht des Griffbereichs des in Figur 1 dargestelten Instruments mit angesetztem Schaltelement;
- Figur 8:: eine perspektivische Ansicht des Instruments aus Figur 1 mit daran angesetztem Schaltelement; und
- Figur 9:: eine Seitenansicht des Instruments aus Figur 1 mit daran angesetztem Schaltelement.

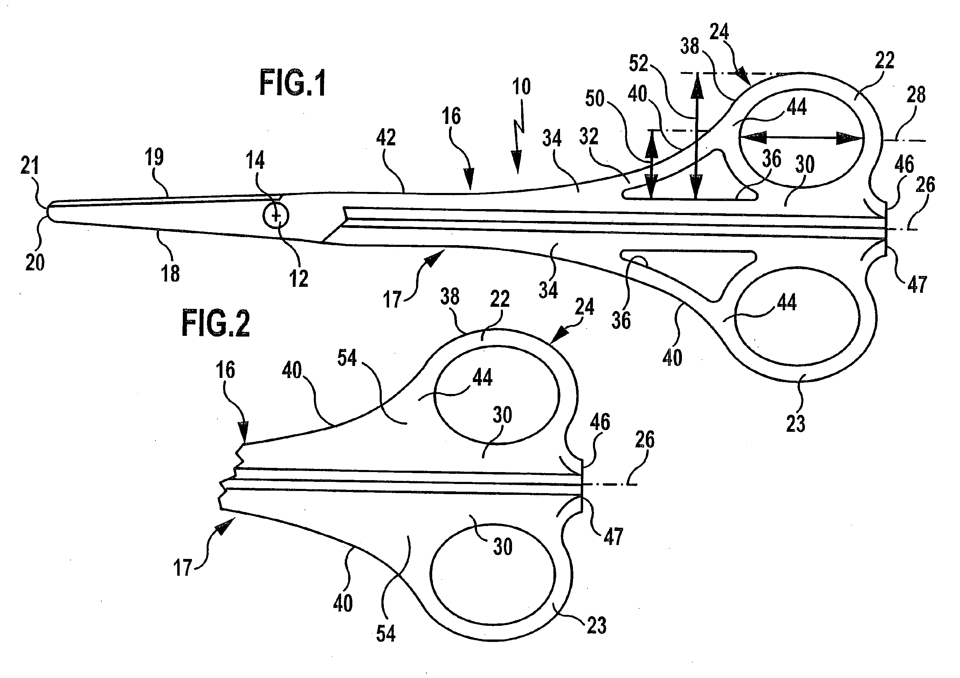

In Figur 1 ist eine insgesamt mit dem Bezugszeichen 10 versehene Bipolarschere dargestellt, die zwei mit einer Lagerschraube 12 verbundene, um eine durch die Lagerschraube 12 definierte Schwenkachse 14 relativ zueinander schwenkbare Branchen 16 und 17 umfaßt. Distalseitig der Lagerschraube 12 setzen sich die Branchen 16 und 17 jeweils in gekrümmten Scherenblättern 18 und 19 fort, die abgerundete Enden 20 und 21 aufweisen.

An ihren proximalen Enden tragen die Branchen 16 und 17 einen Haltering 22 beziehungsweise 23, welche im wesentlichen oval geformt sind. Sie bilden einen Teil eines insgesamt mit dem Bezugszeichen 24 versehenen Griffbereichs der Schere 10. Im Griffbereich 24 sind beide Branchen 16 und 17 symmetrisch zu einer Längsachse 26 der Schere 10 ausgebildet. Nachfolgend wird daher nur der Griffbereich 24 einer der beiden Branchen 16 und 17 exemplarisch beschrieben.

Der ovale Haltering 22 weist eine lange Halbachse 28 auf, die parallel zur Längsachse 26 verläuft. Damit geht der Haltering 22 in einem Verbindungsbereich 30 tangential in die Branche 16 über. Ferner ist ein Verbindungselement in Form eines Stegs 32 vorgesehen, der den Haltering 22 distalseitig mit der Branche 16 verbindet. Er erstreckt sich in etwa von einem distalseitigen Schnittbereich der langen Halbachse 28 und des Halterings 22 in Richtung auf das distale Ende der Branche 16 hin und geht tangential in einem Übergangsbereich 34 in die Branche 16 über. Der Abstand 50 des Übergangsbereichs 44 von der Branche 16 ist in etwa halb so groß wie der größte Abstand 52 der Ringfläche 38 von der Branche 16. Eine Querschnittsfläche des Stegs 32 entspricht im wesentlichen einer Querschnittsfläche des Halterings 22. Eine im wesentlichen dreieckige Durchbrechung 36 mit abgerundeten Ecken wird begrenzt vom Steg 32, der Branche 16 zwischen dem Übergangsbereich 34 und dem Verbindungsbereich 30 und dem Haltering 22.

Eine von der Branche 16 weg weisende Ringfläche 38 des Halterings 22 geht tangential in eine von der Branche 16 weg weisende Haltefläche 40 des Stegs 32 über, welche wiederum in eine Außenfläche 42 der Branche 16 übergeht. Einen minimalen Krümmungsradius weist die Haltefläche 40 in einem Übergangsbereich 44 zwischen dem Haltering 22 und dem Steg 40 auf. Durch den tangentialen Übergang des Stegs 40 in die Branche 16 nimmt der Krümmungsradius der Haltefläche 40 und damit auch des Stegs 32 ausgehend vom Übergangsbereich 44 bis ins Unendliche zu.

Proximale Enden der Brachen 16 und 17 sind mit Buchsen 46 und 47 versehen, die in Form einer Ausnehmung mit sechseckigem Querschnitt ausgebildet sind. Zentral in den Buchsen 46 und 47 angeordnet sind elektrisch leitfähige Kontakte 48 und 49, deren Längsachsen parallel zur Längsachse 26 verlaufen.

In Figur 2 ist eine alternative Ausgestaltung des Griffbereichs 24 der Bipolarschere 10 dargestellt. Er entspricht im wesentlichen dem im Zusammenhang mit der Figur 1 beschriebenen Griffbereich 24. Er unterscheidet sich lediglich dadurch, daß keine Ausnehmung 36 vorgesehen, sondern diese geschlossen und daher ein vollständig geschlossener Stützbereich 54 ausgebildet ist.

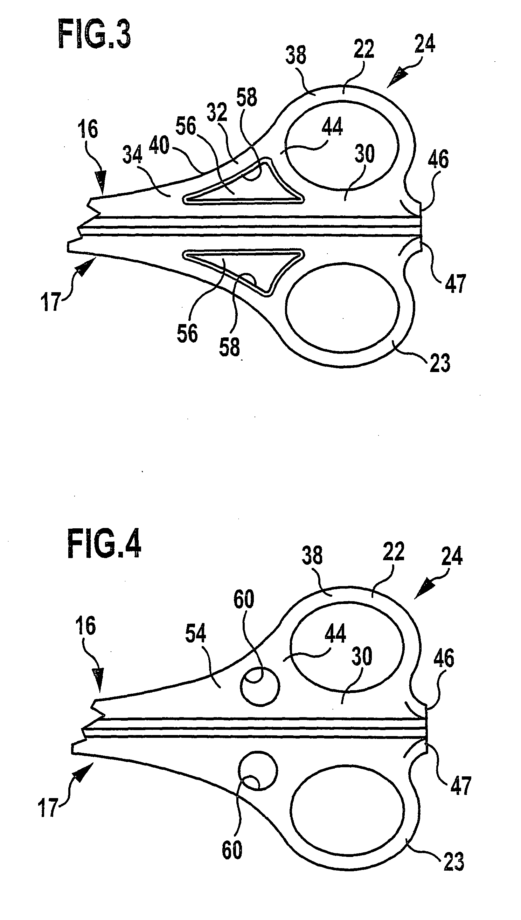

Eine weitere alternative Ausgestaltung eines Griffbereichs 24 ist in Figur 3 dargestellt. Er entspricht im wesentlichen dem Griffbereich 24 der in Figur 1 dargestellten Bipolarschere. Anstelle der Durchbrechung 36 ist jedoch nur eine flache Vertiefung 56 gleicher Form vorgesehen, die ein umlaufender Rand 58 umgibt. Der Rand 58 wird gebildet durch den Steg 32 zwischen den Übergangsbereichen 44 und 34, die Branche 16 zwischen dem Übergangsbereich 34 und dem Verbindungsbereich 30 sowie dem Haltering 22 zwischen dem Verbindungsbereich 30 und dem Übergangsbereich 44.

Eine vierte Alternative eines Griffbereichs 24 ist in Figur 4 dargestellt. Er entspricht im wesentlichen dem in Zusammenhang mit Figur 2 dargestellten Griffbereich 24. Allerdings weist der dort geschlossene Stützbereich 54 zusätzlich eine Bohrung 60 mit abgerundeten Kanten auf.

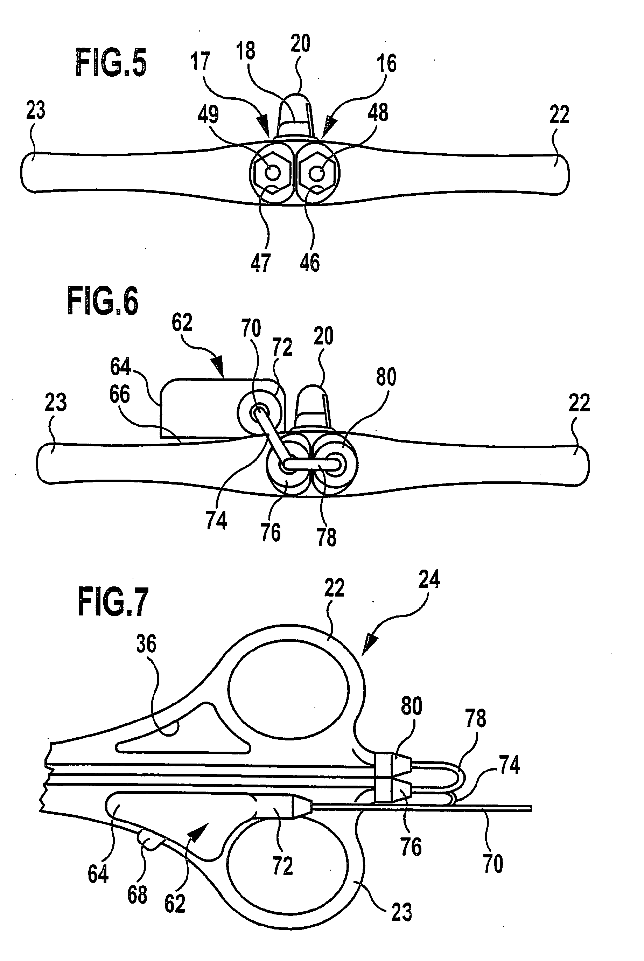

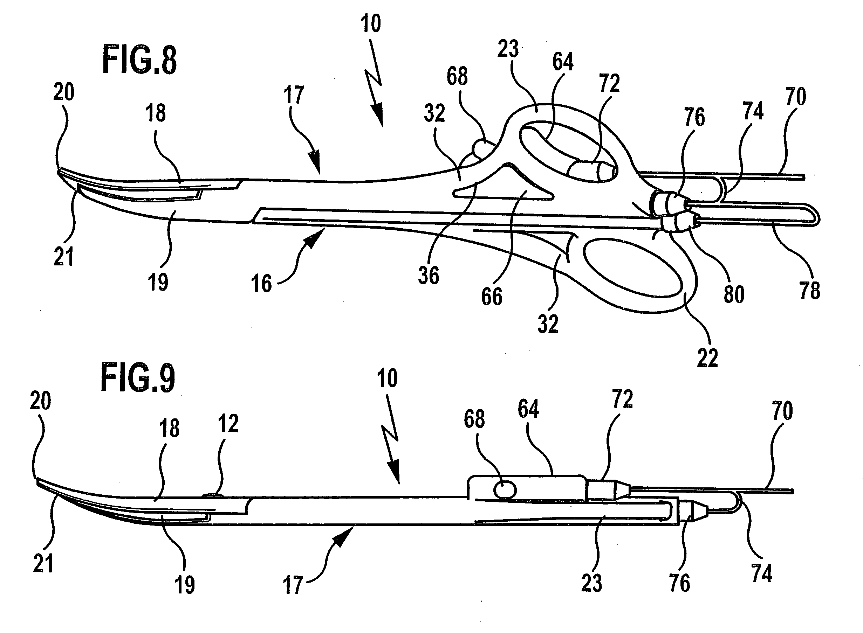

Zum Anschluß der Bipolarschere 10 an eine elektrische Energieversorgung und zum Schließen beziehungsweise Unterbrechen eines Hochfrequenzstromkreises ist an die Bipolarschere 10, wie in Figur 6 dargestellt, ein Schalter 62 anordenbar. Dieser weist ein Gehäuse 64 auf, von dem ein Vorsprung 66 absteht, welcher, wie in Figur 8 dargestellt, korrespondierend zur Durchbrechung 36 ausgebildet ist. Denkbar wäre es auch, den Vorsprung 66 korrespondierend zur Vertiefung 56 oder zur Bohrung 60 auszubilden. Der Vorsprung 66 läßt sich in die Durchbrechung 36 einführen und bei Bedarf zusätzlich durch nicht dargestellte Rastmittel, beispielsweise Zapfen und korrespondierende Ausnehmungen, oder einfach nur aufgrund eines Klemmsitzes mit der Bipolarschere 10 verbinden. Zum Betätigen des Schalters 62 ist dieser mit einem seitlich abstehenden Schaltknopf 68 versehen.

Zur einfachen Verbindung der Bipolarschere 10 mit einem nicht dargestellten Netzgerät ist eine Anschlußleitung 70 über einen Stecker 72 mit dem Schalter 62 verbunden. Aus dem Stecker 72 herausgeführt ist eine Verbindungsleitung 74, die zu einem korrespondierend zur Buchse 47 ausgebildeten Stecker 76 führt. Von diesem geht eine weitere Verbindungsleitung 78 zu einem dritten Stecker 80, der korrespondierend zur Buchse 46 ausgebildet und in dieser ebenso verdrehgesichert ist, wie der Stecker 76 in der Buchse 47.

Die im Zusammenhang mit den Figuren 1 bis 9 beschriebenen Buchsen 46 und 47 sind mit beliebigen Scherenformen und beliebig geformten Griffbereichen 24 kombinierbar, insbesondere auch mit den in den Figuren 1 bis 4 dargestellten Griffbereichen 24. Ebenso kann der Schalter 62 unabhängig von der Ausgestaltung der Griffbereiche 24 mit einer Bipolarschere 10 oder einem anderen Instrument verbunden werden. Insbesondere wäre es möglich, die Buchsen 46 und 47 an einem Rohrschaftinstrument vorzusehen, ebenso Schaltelementaufnahmen in Form der Durchbrechung 36, der Bohrung 60 oder der Vertiefung 56.

Ferner kommt es bei der Ausgestaltung der Griffbereiche 24 nicht auf die weitere Ausgestaltung des Instruments an. Werkzeuge der Instrumente können beliebig ausgebildet sein.

## Patentansprüche

1. Chirurgisches Instrument mit mindestens zwei aneinander oder relativ zueinander bewegbaren Branchen zum Betätigen von Werkzeugen an einem distalen Ende des Instrumentes, wobei die Branchen jeweils einen in einem proximalen Bereich der Branchen abstehenden Haltering tragen, **dadurch gekennzeichnet, daß** mindestens eine der beiden Branchen (16, 17) ein Verbindungselement (32; 54) umfaßt zum Herstellen einer zusätzlichen Verbindung des Halterings (22; 23) mit der Branche (16, 17) und daß das Verbindungselement (32; 54) eine von der Branche (16, 17) weg weisende Haltefläche (40) umfaßt.

2. Instrument nach Anspruch 1, **dadurch gekennzeichnet, daß** die Halteringe (22, 23) der beiden Branchen (16, 17) seitlich von den Branchen (16, 17) abstehen und eine gemeinsame Ebene definieren.

3. Instrument nach einem der voranstehenden Ansprüche, **dadurch gekennzeichnet, daß** das Verbindungselement (32; 54) tangential in den Haltering (22, 23) übergeht.

4. Instrument nach einem der voranstehenden Ansprüche, **dadurch gekennzeichnet, daß** die Haltefläche (40) tangential in eine von der anderen Branche (17) weg weisende Fläche (42) der Branche (16) übergeht.

5. Instrument nach einem der voranstehenden Ansprüche, **dadurch gekennzeichnet, daß** das Verbindungselement (32; 54) tangential in die Branche (16, 17) übergeht.

6. Instrument nach einem der voranstehenden Ansprüche, **dadurch gekennzeichnet, daß** die Haltefläche (40) tangential in eine radial nach außen weisende Fläche (38) des Halterings (22, 23) übergeht.

7. Instrument nach einem der voranstehenden Ansprüche, **dadurch gekennzeichnet, daß** der Krümmungsradius der nach außen weisenden Haltefläche (40) mindestens 1 cm beträgt.

8. Instrument nach einem der voranstehenden Ansprüche, **dadurch gekennzeichnet, daß** die Haltefläche (40) sich vom Haltering (22, 23) in distaler Richtung der Branche (16, 17) erstreckt.

9. Instrument nach einem der voranstehenden Ansprüche, **dadurch gekennzeichnet, daß** die Haltefläche (40) am Haltering (22, 23) ansetzt in einem Abstand (50) von der Branche (16), der etwa der halben Quererstreckung (52) des Halterings (22) quer zur Längsrichtung (26) der Branche (16) entspricht.

10. Instrument nach einem der voranstehenden Ansprüche, **dadurch gekennzeichnet, daß** die Haltefläche (40) einen von dem Haltering (22) zunehmenden Krümmungsradius aufweist.

11. Instrument nach einem der voranstehenden Ansprüche, **dadurch gekennzeichnet, daß** das Verbindungselement einen Stützsteg (32) umfaßt.

12. Instrument nach Anspruch 11, **dadurch gekennzeichnet, daß** der Stützsteg (32) eine Querschnittsfläche aufweist, die im wesentlichen der Querschnittsfläche des Halterings (22) entspricht.

13. Instrument nach einem der Ansprüche 1 bis 10, **dadurch gekennzeichnet, daß** das Verbindungselement einen von dem Haltering (22), der Branche (16) und der Haltefläche (40) umgebenen Stützbereich (54) ausfüllt.

14. Instrument nach einem der voranstehenden Ansprüche, **dadurch gekennzeichnet, daß** das Verbindungselement (32; 54) eine Ausnehmung (56) oder Durchbrechung (36; 60) umfaßt.

15. Instrument nach Anspruch 14 , **dadurch gekennzeichnet, daß** die Ausnehmung (56) oder Durchbrechung (36; 60) im wesentlichen rund oder dreieckig ausgebildet ist.

16. Instrument nach einem der voranstehenden Ansprüche, **dadurch gekennzeichnet, daß** die Branchen (16, 17) aneinander schwenkbar gelagert sind und daß die Werkzeuge (18, 19) starr mit den Branchen (16, 17) verbunden sind.

17. Instrument nach dem Oberbegriff des Anspruchs 1 oder nach einem der voranstehenden Ansprüche, **dadurch gekennzeichnet, daß** das Instrument (10) eine elektrische Anschlußvorrichtung (46, 47) umfaßt.

18. Instrument nach Anspruch 17, **dadurch gekennzeichnet, daß** die elektrische Anschlußvorrichtung (46, 47) eine Verdrehsicherung umfaßt zum Verhindern eines Verdrehens eines mit der Anschlußvorrichtung (46, 47) verbundenen Anschlußelementes (76, 80).

19. Instrument nach Anspruch 18, **dadurch gekennzeichnet, daß** die Verdrehsicherung eine mindestens einen Anschlußkontakt (48, 49) umgebende Ausnehmung (46, 47) umfaßt und daß die Ausnehmung (46, 47) einen vieleckigen Querschnitt, insbesondere einen 6-eckigen Querschnitt aufweist.

20. Instrument nach Anspruch 19, **dadurch gekennzeichnet, daß** der mindestens eine Anschlußkontakt (48, 49) in eine zur Längsrichtung (26) der Branche (16, 17) parallele Richtung weist.

21. Instrument nach dem Oberbegriff des Anspruchs 1 oder nach einem der voranstehenden Ansprüche, **dadurch gekennzeichnet, daß** das Instrument (10) ein Schaltelement (62) umfaßt zum Unterbrechen eines Energieflusses von einer Energiequelle zum Instrument (10).

22. Instrument nach Anspruch 21, **dadurch gekennzeichnet, daß** das Schaltelement (62) mit dem Instrument (10) lösbar verbindbar ist.

23. Instrument nach einem der Ansprüche 21 oder 22, **dadurch gekennzeichnet, daß** das Schaltelement (62) mit dem Instrument (10) verrastbar oder an dieses anclipbar ist.

24. Instrument nach einem der voranstehenden Ansprüche, **dadurch gekennzeichnet, daß** das Instrument (10) eine Schaltelementaufnahme (36; 56; 60) umfaßt.

25. Instrument nach Anspruch 24, **dadurch gekennzeichnet, daß** die Schaltelementaufnahme durch die Ausnehmung (56) oder Durchbrechung (36; 60) des Verbindungselementes (32; 54) gebildet ist.

26. Instrument nach einem der voranstehenden Ansprüche, **dadurch gekennzeichnet, daß** die Werkzeuge Klemmbacken oder Scherenblätter (18, 19) mit Schneiden umfassen.

27. Instrument nach einem der voranstehenden Ansprüche, **dadurch gekennzeichnet, daß** der Haltering (22, 23) rund oder oval rechteckig ist.

28. Instrument nach einem der voranstehenden Ansprüche, **dadurch gekennzeichnet, daß** das Instrument (10) aus Kunststoff hergestellt ist.

29. Instrument nach einem der voranstehenden Ansprüche, **dadurch gekennzeichnet, daß** die Branche (16, 17), der Haltering (22, 23) und das Verbindungselement (32; 54) einstückig ausgebildet sind.
